# EUROPEAN PATENT SPECIFICATION

(11) **EP 2 296 743 B1**
(45) Date of publication and mention of the grant of the patent: **08.04.2020**
(21) Application number: 09750184.5
(22) Date of filing: 21.05.2009
(51) Int. Cl.: A61M 25/00

(54) **INJECTION DEVICE**
INJEKTIONSVORRICHTUNG
DISPOSITIF D'INJECTION

(30) Priority: 22.05.2008 GB 0809361
(43) Date of publication of application: 23.03.2011
(73) Proprietor: TiGenix, S.A.U., 28760 Tres Cantos, Madrid (ES)
(72) Inventor: PUGH, Mark, Co. Sligo (IE); ZANOTTI, Carlo, E-28760 Tres Cantos, Madrid (ES); OROFINO, Javier, E-28760 Tres Cantos, Madrid (ES); MEDINA, Jesus, E-28760 Tres Cantos, Madrid (ES)
(74) Representative: Lasar, Andrea Gisela
(86) International application number: PCT/IB2009/005827
(87) International publication number: WO 2009/141727

(56) References cited:
- EP-A- 1 078 644
- WO-A-01/89605
- WO-A-97/41786
- WO-A-03/094855
- WO-A-2007/051563
- US-A1- 2003 069 547
- US-A1- 2006 041 243

## Description

### Field of the Invention

The present invention relates to an injection device. More specifically, the present invention relates to an injection device for delivering fluid to an injection site, for example delivering cells into the walls of vessels in tissue.

### Background of the Invention

It is known to use injection devices for subcutaneous injections. These devices conventionally comprise a housing and a syringe with a drive mechanism that moves the syringe into an extended position and which then ejects fluid from a needle connected to the syringe.

See, e.g., WO 2007/051563 A.

The problem with these conventional devices is that there has to be at least two independent actions. The syringe contains the fluid to be injected and this must first be moved within the injection device to extend the needle out of the injection device. The second action is the ejection of fluid from the needle once the syringe and needle have reached their extended positions. Having two separate actions makes the device complex and it is difficult to achieve accurate placement of the needle. This is particularly the case when injecting into locations that cannot be seen by the user, for example into walls of vessels or pathways, e.g. a vessel wall, in particular, into the wall of a track in an enterocutaneous fistula or into the rectal mucosa in the case of rectovaginal and perianal fistulas.

In general terms, a fistula is an abnormal connection or passageway between organs or vessels that normally do not connect. Fistulae can develop in various parts of the body. For example, types of fistulae, named for the areas of the body in which they occur, include anorectal fistula or fistula-in-ano or fecal fistula (between the rectum or other anorectal area and the skin surface), arteriovenous fistula or A-V fistula (between an artery and vein), biliary fistula (between the bile ducts to the skin surface, often caused by gallbladder surgery), cervical fistula (abnormal opening in the cervix), craniosinus fistula (between the intracranial space and a paranasal sinus), enteroenteral fistula (between two parts of the intestine), enterocutaneous fistula (between the intestine and the skin surface, namely from the duodenum or the jejunum or the ileum), enterovaginal fistula (between the intestine and the vagina), gastric fistula (between the stomach to the skin surface), metroperitoneal fistula (between the uterus and peritoneal cavity), perilymph fistula (a tear between the membranes between the middle and inner ears), pulmonary arteriovenous fistula (between an artery and vein of the lungs, resulting in shunting of blood), rectovaginal fistula (between the rectum and the vagina), umbilical fistula (between the umbilicus and gut), tracheoesophageal fistula (between the breathing and the feeding tubes) and vesicovaginal fistula (between the bladder and the vagina). Causes of fistulae include trauma, complications from medical treatment and disease.

It has become apparent that fistulae can be treated with an injection of stem cells. Cell populations and compositions that might be used are disclosed in Garcia-Olmo et al. (Int J Colorectal Dis 2003, 18:451-454) and Garcia-Olmo et al. (Dis Colon Rectum 2005, 48:1416-1423), and in the international patent applications published as WO 2006/037649, WO 2006/136244, WO 2007/039150 and WO 2007/065927.

Injection of stem cells into fistulae or into walls of vessels which are connected by fistulae requires very accurate placement of the injection needle to the target injection site. This is difficult to achieve when relying on a conventional syringe and plunger. Moreover, since the cells are being injected internally into tissue, it is difficult to achieve accurate placement via narrow delivery pathways without damaging the cells.

### Summary of the Invention

The present invention aims to solve the aforementioned problems.

The invention is defined by the appended claims. Subject-matter referred to as embodiments and/or inventions not being under the scope of the appended claims merely represent possible exemplary executions and are not part of the invention.

It is an aim of the present invention to locate accurately a target injection site with an injection device and provide injection of an amount of fluid to the target injection site.

The invention provides an injection device comprising:
a housing adapted to receive a fluid container and hold the fluid container stationary relative to the housing during use of the device; and
a discharge nozzle movable between a first retracted position in which the discharge nozzle is contained within the housing and a first extended position in which the discharge nozzle has moved relative to the fluid container and extends from the housing through an exit aperture of the housing,
wherein the fluid container and discharge nozzle are in fluid communication with each other.

The fluid container and discharge nozzle are in fluid communication with each other in the first extended position, in the first retracted position and in all positions therebetween.

Holding the fluid container stationary relative to the housing during use of the injection device provides a number of advantages. For example, extension of the needle and expulsion of a predetermined and fixed quantity of fluid can be achieved in a single continuous action which is smooth and which does not cause movement of the injection needle at the target injection site. Moreover, a single action means that the device is less complex and it is easier to achieve accurate placement of the needle. In addition, rather than having to move the entire syringe assembly to the target injection site, only the discharge nozzle, e.g. needle, is moved to the injection site which allows for much more accurate needle placement.

The injection device further comprises a plunger, wherein the plunger is adapted to discharge fluid from the fluid container.

The injection device further comprises a drive, wherein the drive is adapted to advance the discharge nozzle from its first retracted position to its first extended position. The drive is adapted to act upon the plunger to expel fluid from the fluid container through the discharge nozzle.

The drive is adapted such that only when the discharge nozzle has reached its first extended position, does the drive act upon the plunger to expel fluid from the fluid container through the discharge nozzle. This ensures that fluid is only delivered to a target injection site when the discharge nozzle, e.g. needle, has been extended sufficiently for injection of the fluid to take place.

Optionally, the drive is adapted to act upon the discharge nozzle and the plunger such that the discharge nozzle and plunger each move a predetermined distance in a single continuous motion. As mentioned above, having a single action makes the device less complex and it is easier to achieve accurate placement of the discharge nozzle and hence fluid.

The plunger comprises a serrated ratchet.

The drive comprises a first member and a second member, wherein the second member comprises a resiliently biased member adapted to engage the ratchet at a predetermined position of the drive relative to the housing. The fixed separation of the ratchet slots on the plunger provides for injection of a predetermined quantity of fluid each time the plunger is advanced from its retracted position. After each injection, the drive is withdrawn to its retracted position, whilst the plunger remains in the position it reached in the fluid container, ready to be further inserted to inject a further fixed quantity of fluid.

Preferably, the housing further comprises a first sleeve, wherein the discharge nozzle is contained within the first sleeve when in its first retracted position, and wherein the discharge nozzle extends from the first sleeve when in its first extended position.

Optionally, the housing comprises a second sleeve, wherein the second sleeve and the discharge nozzle are movable between a second retracted position in which the second sleeve and the discharge nozzle are contained within the first sleeve and a second extended position in which the second sleeve and the discharge nozzle extend from the first sleeve through an exit aperture in the first sleeve.

Preferably, when the second sleeve is in its second extended position, the discharge nozzle is movable between a third retracted position in which the discharge nozzle is contained within the second sleeve and its first extended position in which the discharge nozzle extends from the second sleeve through an exit aperture in the second sleeve. The second sleeve is moveable relative to the discharge nozzle so that there is an indication on whether or not the discharge nozzle has been inserted into tissue. If it has, injection can take place. This is achieved because the sliding sleeve does not enter tissue when the discharge nozzle is extended if tissue is present and impedes the movement of the sleeve, whereas the discharge nozzle can pass into the tissue, thereby extending beyond the end of the second sleeve. The relative displacement of the second sleeve and discharge nozzle can then be detected to give an indication of whether the discharge nozzle has entered tissue. Alternatively, the second sleeve can be connected to a locking mechanism which prevents fluid being expelled from the fluid container.

The injection device may comprise a biasing means that is adapted to couple the second sleeve to the drive.

The injection device may comprise a releasable stop means for releasably stopping the plunger after it has been moved a predetermined distance.

Optionally, the releasable stop means comprises a serrated member for interlocking with the serrated ratchet. Advantageously, the stop means is moveable on application of force by a user between an engaged position in which it engages with the serrated ratchet, preventing movement of the ratchet towards the proximal end of the injection device, and a disengaged position in which the stop means does not engage with the serrated ratchet, thereby permitting the plunger to be withdrawn towards the proximal end of the injection device. Even in its engaged position, the plunger can still move towards the distal end of the injection device, but cannot move towards the proximal end. This means that the plunger is maintained in a fixed position within the fluid container after a predetermined quantity of fluid has been ejected. Retraction of the drive and discharge nozzle does not result in the plunger being moved. This way, the injection device can be moved to another target injection site for injection of a further predetermined quantity of fluid.

Preferably, the fluid container comprises a cannular body, a flange at one end of the body and a discharge nozzle at a distal end of the body.

The injection device may comprise a flexible fluid pathway between the fluid container and the discharge nozzle. Preferably, the flexible fluid pathway is located adjacent to the fluid container. The flexible pathway may be adapted to straighten and bend, or extend and contract such that the discharge nozzle is movable between its first retracted position and its first extended position. The flexible fluid pathway may be manufactured as a coil of hollow tubing, advantageously a helical coil, which can extend and contract as the discharge nozzle moves relative to the fluid container. The coil may be formed from a metal, advantageously a resilient, flexible metallic material.

Preferably, the injection device comprises a connection element between the discharge nozzle and the drive, or between the flexible fluid pathway and the drive, such that when the drive is activated the discharge nozzle moves from its first retracted position to its first extended position.

Optionally, a stop means is provided for stopping the drive.

Preferably, the first sleeve comprises a cannular catheter.

Preferably, the drive comprises a depressible button.

Preferably, when the discharge nozzle has reached its first extended position, it continues to extend a predetermined distance as the drive is moved.

Preferably, when the discharge nozzle is at its first extended position, the discharge nozzle is at an angle to the first sleeve. In this way, the discharge nozzle is flexible so that it can exit the first sleeve at a plurality of points along the first sleeve. Advantageously, it is easier to achieve accurate placement of the needle when the needle is flexible.

Optionally, a distal end of the first sleeve comprises an aperture and a suture anchor.

In an example provided as part of the present disclosure, there is provided an injection device comprising:
a housing;
a fluid container in the housing;
a discharge nozzle, connected to an end of the fluid container, through which fluid can be ejected;
a user-actuatable drive for causing ejection of the fluid from the fluid container; and
a sleeve surrounding at least a portion of the discharge nozzle,
wherein the discharge nozzle is movable between a first retracted position in which the discharge nozzle is contained within the housing, and a first extended position in which the discharge nozzle extends from the housing through an exit aperture in the housing,
wherein the sleeve is adapted to move with the discharge nozzle as the discharge nozzle is being extended into its first extended position, and the sleeve is further adapted to move a predetermined distance relative to the discharge nozzle to expose a section of the discharge nozzle from an end of the sleeve.

Preferably, the injection device comprises an indicator which is viewable by a user of the injection device and which is adapted to indicate that fluid can be ejected from the fluid container only when the sleeve has moved the predetermined distance.

Alternatively, the injection device comprises a locking mechanism which is connected to the sleeve and drive and which is adapted to permit the drive to be moved to eject fluid from the fluid container only when the sleeve has moved the predetermined distance. In this way, ejection of the fluid will not place when the discharge nozzle is located in a cavity and not in tissue.

Preferably, the sleeve and discharge nozzle move relative to each other only when the sleeve and discharge nozzle come into contact with tissue, such that the discharge nozzle enters the tissue and the sleeve does not enter the tissue. In this way, ejection of fluid will only take place when the discharge nozzle has entered the tissue.

Preferably, the discharge nozzle is a needle and/or the fluid container is a syringe.

The discharge nozzle preferably has an internal diameter which is no less than (i.e. greater than or equal to) 0.4 mm along its length, or more preferably no less than (i.e. greater than or equal to) 0.5 mm along its length and advantageously may be no less than (i.e. greater than or equal to) 0.6, 0.7, 0.8, 0.9 or 1.0 mm along its length. Such a diameter is advantageous in preventing breakage of cells which might be injected from the injection device. Cells ejected from nozzles having smaller diameters than 0.5 mm, run the risk of being damaged by the increased pressure through the nozzle due to the reduced diameter and the friction between the cells and the internal wall of nozzle. The outer diameter of the needle is preferably greater than or equal to 0.9 mm, more preferably greater than or equal to 1.5 mm.

In another aspect of the present invention, there is provided a kit, comprising the injection device and a fluid container comprising a population of cells.

In one embodiment of the present invention, the fluid container comprises a population of human or animal cells. In another embodiment of the present invention, the fluid container comprises a population of stem cells.

Preferably, the cells are present in the fluid container in the form of a composition comprising the cells and a pharmaceutically acceptable carrier or excipient.

### Brief Description of Drawings

One or more embodiments of the present invention are described below with reference to the accompanying drawings, in which:-
Fig. 1 shows a perspective view of the injection device of the present invention;
Fig. 2 shows a top plan view of the injection device of Fig. 1;
Fig. 2a shows a further top plan view of the injection device of Fig. 1;
Fig. 3 shows a right side cross-sectional view of the injection device of Fig. 1;
Fig. 4a is an enlarged side view of a section of the injection device of Fig. 1;
Fig. 4b is a further enlarged side view of a section of the injection device of Fig. 1;
Fig. 5a is a perspective view of an enlarged section of a catheter of the injection device of Fig. 1;
Fig. 5b is a cross-sectional view of the catheter of Fig. 5a;
Fig. 5c is a further cross-sectional view of the catheter of Fig. 5a;
Fig. 5d is a further cross-sectional view of the catheter of Fig. 5a; and
Fig. 6 is a side view of an embodiment of a fluid pathway implemented in the injection device of Fig. 1.

### Detailed Description of the Drawings

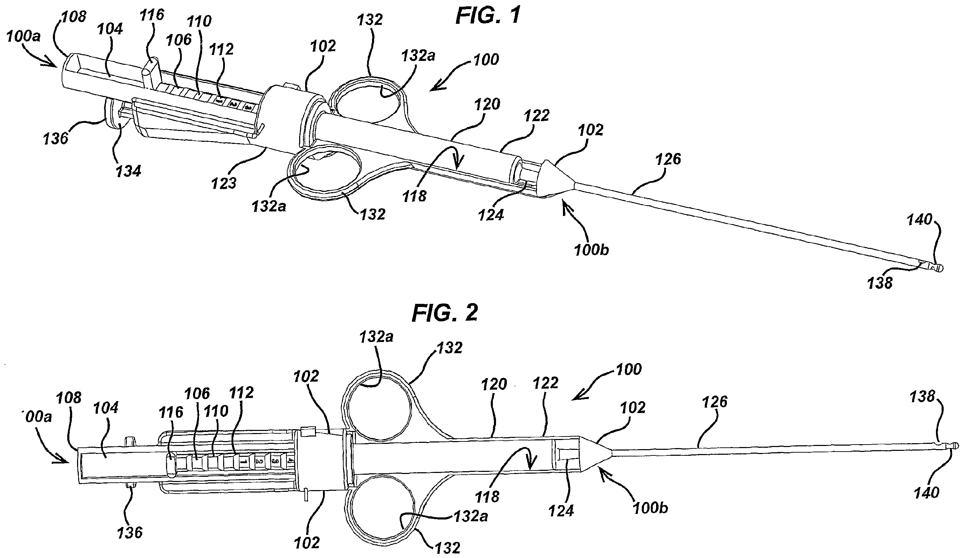

With reference to Figs. 1 to 3, there is illustrated an injection device 100 according to one embodiment of the present invention. The injection device 100 has a proximal end 100a and a distal end 100b. A syringe 120 is held in the injection device towards its distal end 100b.

The injection device 100 comprises a housing 102 and a channel 104 that is shaped and dimensioned to receive a plunger 106. The channel 104 comprises an end wall 108 at the proximal end 100a for restricting movement of the plunger 106 out of the proximal end of the channel 104. At its distal end, the channel is open to provide a pathway to the syringe 120. The plunger 106 sits in the channel and can slide into an open end of the syringe 120 to act on a bung 119 in the syringe 120.

The plunger 106 comprises a serrated ratchet 110 which comprises a plurality of teeth 112 which are spaced apart from each other by a predetermined distance. The plunger 106 further comprises an end member 116, in the form of a projection which extends out of the channel 104. The end member 116 is for use by a user to locate the plunger 106 in a desired start position within the channel 104 and syringe 120 and for relocating the plunger 106 to its start position. The start position is defined as the position in which the plunger 106 is first positioned by a user to act on fluid in the syringe 120 when a new syringe 120 is inserted into the device 100.

The housing 100 has a slot 118 adjacent to the channel 104 towards the distal end 100b of the injection device 100. The slot 118 is shaped and dimensioned to receive a fluid container, in the present embodiment in the form of a syringe 120. The slot 118 is further shaped and dimensioned to hold the syringe 120 stationary during use of the injection device 100. The syringe 120 comprises a cannular body 122, a flange 123 and an opening dimensioned to receive the plunger at a proximal end 100a of the body 122, and a nozzle 124 at the distal end 100b of the body 122.

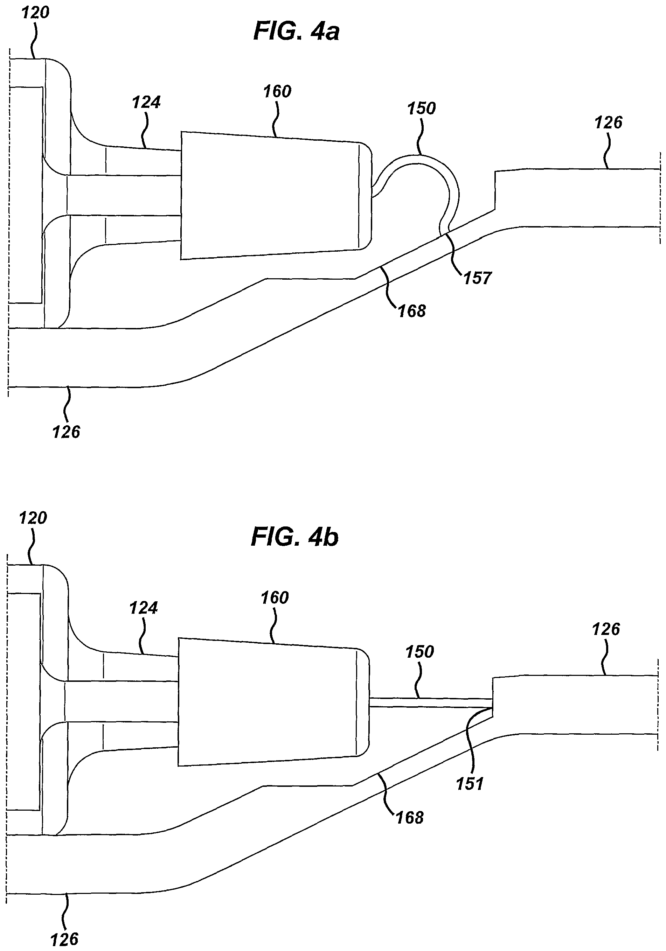

A sleeve is connected to the distal end 100b of the housing 102 in the form of a hollow catheter 126. The catheter 126 is shaped and dimensioned internally to receive an elongated discharge nozzle in the form of a needle 128 (not shown). The catheter 126 has an aperture 138 at its distal end 100b through which the needle 128 projects when moved to an extended position. In a retracted position, the needle 128 does not project from the aperture 138. The distal end of the catheter 126 also comprises a recess/suture anchor 140 in the form of a hole through the catheter 126 for use in suturing. The syringe 120 and the needle 128 are fluidly connected to each other by a flexible fluid pathway. The fluid pathway is described in more detail below in connection with Figs. 4a and 4b.

The housing 102 also comprises gripping means, in the form of a pair of ears 132 which are positioned on an outer surface of the housing 102. Each ear 132 has an aperture 132a which is shaped and dimensioned to enable a user to grip the injection device 100 by placing one of the index and middle finger of one hand through a respective aperture 132a.

A drive 134 is located at the proximal end 100a of the injection device adjacent to the channel 104. The drive 134 is connected to the needle 128 as will be explained below. The drive 134 is actuated by means of a depressible button 136 which can be pressed by a user's thumb on the same hand as his middle and index fingers when each is inserted through a corresponding aperture 132a.

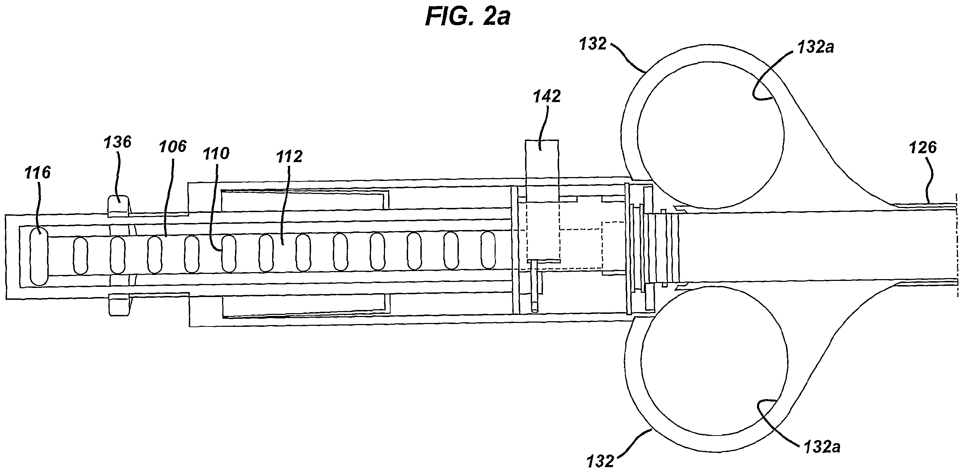

As can be seen in Fig. 2a, the injection device 100 further comprises a releasable stop means such as a stop member 142 with at least one tooth for interlocking with the serrated ratchet 110 of the plunger 106. The stop member 142 is moveable on application of force by a user between an engaged position in which its tooth engages with the serrated ratchet 110, preventing movement of the ratchet towards the proximal end 100a of the injection device 100, and a disengaged position in which the tooth does not engage with the serrated ratchet 110, thereby permitting the plunger 106 to be withdrawn towards the proximal end 100a of the injection device 100. Even in its engaged position, the plunger 106 can move towards the distal end 100b of the injection device 100. The stop member 142 is resiliently biased, for example by a spring, into its engaged position.

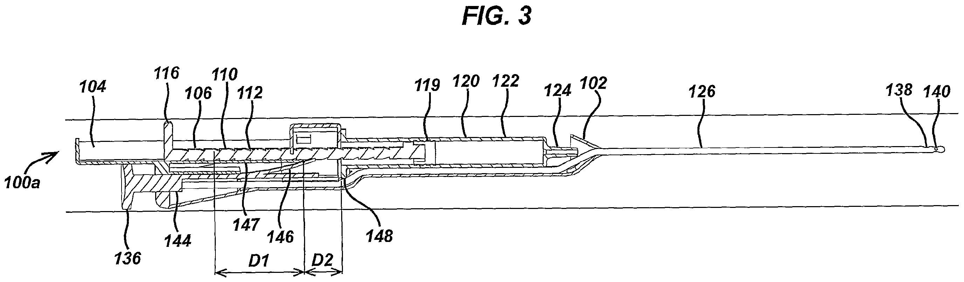

As can be seen in Fig. 3, the drive 134 comprises a first drive member 144 directly connected to the needle 128 and a second drive member 146 that is resiliently biased towards the serrated ratchet 110. A retainer 147 extending from the proximal end 100a towards the distal end 100b is located between the drive 136 and the serrated ratchet 110. The retainer 147 prevents the second drive member 146 from engaging with the serrated ratchet 110 for a predetermined distance i.e. until the needle 128 has reached its extended position, as will be explained in more detail below. A stop 148 is also provided in the injection device 100 at the end of the channel in which the drive moves. The stop 148 prevents movement of the drive 136 beyond a predetermined distance and thus a fixed predetermined quantity of fluid will always be ejected from the syringe which is set by the distance D2 from the end of the retainer to the stop 148.

Figs. 4a and 4b show in more detail how the syringe 120 is connected to the needle 128 at the distal end of the device 100b. The nozzle 124 tapers into a male luer 160. The male luer 160 is connected to the needle 128 in the catheter 126 via the flexible fluid pathway 150. The flexible fluid pathway 150 is connected to the needle 128 by way of a skive 168 in the catheter 126. Fig. 4a shows the needle 128 in its retracted position and Fig. 4b shows the needle 128 in its extended position.

The flexible fluid pathway 150 is adapted to straighten and bend, or extend and contract such that the needle 128 is movable between its retracted position and its extended position. The needle 128 is also flexible or semi-rigid so as to bend through the catheter 126 out of the aperture 138. The needle 128 and flexible fluid pathway 150 are integrably formed with each other. The needle 128 and the flexible fluid pathway are manufactured from PEEK coated fused silica. As PEEK coated fused silica is not as strong as steel, the outer diameter of the needle 128 will be larger than the standard gauge size, for example 1.5mm rather than 0.91mm in a 20 Gauge needle. It is also envisioned that the internal diameter of the needle may be 0.53mm. This is between the 20 Gauge (0.61mm) and 22 Gauge (0.41mm) internal diameter that is used in conventional needles. A connection element (not shown) is located between the needle 128 and the drive 134, or between the flexible fluid pathway 150 and the drive 134, and integrated with them such that when the drive is activated the needle 128 moves from its retracted position to its extended position.

In use, the injection device 100 containing fluid to be injected into a target injection site is placed in a desired location by an operator. For example, this might include placing the catheter 126 inside the rectum and into a fistula tract. Once in the desired location, the button 136 at the proximal end 100a of the injection device 100 is pushed towards the distal end 100b causing the first 144 and second 146 drive members to move. As the first drive member 144 moves, it will cause the needle 128 to move towards the distal end 100b from its retracted position in which the needle 128 is contained within the catheter 126 to its extended position in which the needle 128 has moved relative to the syringe 120 and extends from the catheter 126 through the aperture 138 into tissue. As the second drive member 146 moves, the retainer 147 prevents the second drive member 146 from engaging with the serrated ratchet 110 for a first predetermined distance D1.

Once the drive 134 has moved the first predetermined distance D1, the needle 128 will be in its extended position and the second drive member 146 will no longer be prevented from engaging with the serrated ratchet 110. As the drive 134 continues to move from a first predetermined position to a second predetermined position, the second drive member 146 engages with a least one tooth 112 of the serrated ratchet 110 causing the plunger 106 to move a predetermined distance D2 from a start position along the channel 104 in the syringe 120 which in turn causes the bung 119 of the syringe 120 to move into the syringe 120 to expel a predetermined volume of the fluid into the target injection site. Once the drive 134 has moved the second predetermined distance D2, the stop 148 stops the drive 134 from ejecting more fluid than that which is set by the second predetermined distance D2. The stop member 142 automatically engages with the serrated ratchet 110 on the plunger 106, preventing movement of the ratchet 110 towards the proximal end 100a of the injection device 100.

In preparation for the next injection, the button 136 is pulled back towards the proximal end 100a which causes the needle 128 to return to its retracted position. The injection device 100 is now ready to deliver another predetermined volume of fluid. However, because the stop member 142 stops the plunger 106 from moving when the button 136 is pulled back towards the proximal end 100a, the plunger 106 is maintained at the predetermined distance D2 from its previous start position. The above-described steps of injecting predetermined volumes of fluid can be repeated until the syringe 128 has expelled its last remaining predetermined volume of fluid. At this time, or when injections for a particular patient have been completed, the stop member 142 can be actuated to release it from the plunger 106, thereby permitting the plunger 106 to be retracted out of the syringe 128. The syringe 120 can then be replaced or refilled and the plunger 106 can be relocated to its start position so that the injection device 100 is ready to inject a predetermined amount of fluid once more.

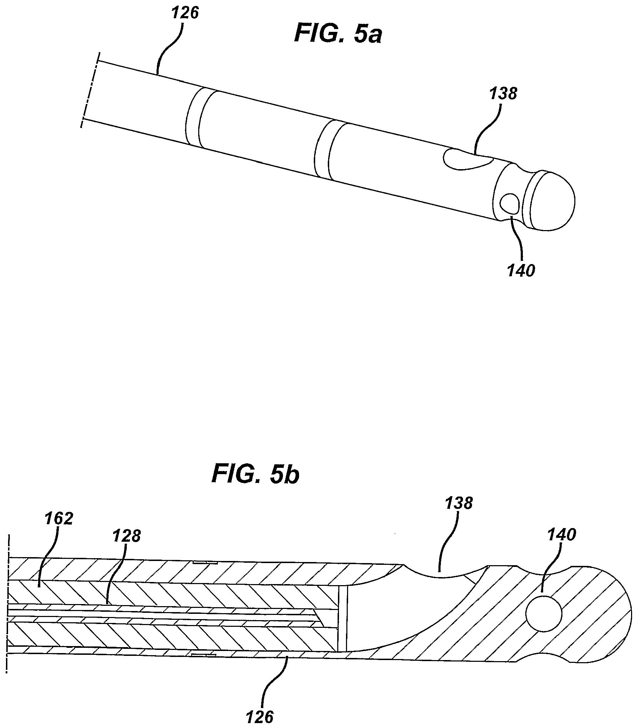

Figs. 5a to 5d show a particular embodiment of needle 128 and catheter 126 of the injection device 100 of the present invention.

Fig. 5a shows a perspective view of the end of the catheter 126 with the aperture 138 which allows the needle 128 and a feeler tube 162 to pass though.

Fig. 5b is a cross-section of the catheter 126 of Fig. 5a showing needle 128 and the feeler tube 162 retracted from the aperture 138. This is the resting position of the injection device 100.

When the button 136 is pressed, the needle 128 will advance as described above in connection with Figs. 1 to 4b. In this particular embodiment, the feeler tube 162 is mounted around the needle 128, and is resiliently biased, for example by a spring (not shown) located at the distal end 100b of the housing 102, to enable the feeler tube 162 to move with the needle 128 as the needle 128 is being extended into its extended position, the feeler tube 162 moves a predetermined distance relative to the needle 128 to expose a section of the needle 128 from an end of the feeler tube 162, and the drive 134 is adapted to eject fluid from the syringe 120 and needle 128 as described above only when the feeler tube 162 has moved the predetermined distance. Alternatively, the relative position of the feeler tube 162 with respect to the needle 128 can be viewed by a user through the housing In other words, the needle 128 is movable between a locked position in which the needle 128 is contained within the feeler tube 162 and an unlocked position in which the needle 128 extends a predetermined distance from the feeler tube 162. A locking mechanism (not shown) can be provided to act upon the drive 134 to prevent the second drive member 146 from engaging with the serrated ratchet 110 when the needle 128 is in its locked position; thereby preventing expulsion of fluid when the needle 128 is in its locked position.

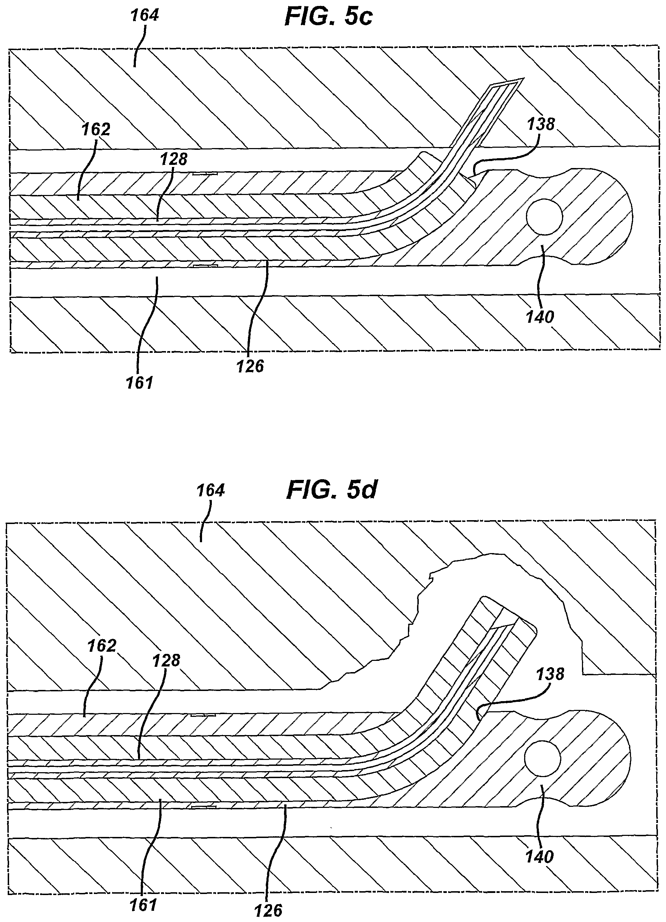

As can be seen in Fig. 5c, the catheter 126 has been placed in a fistula tract 161. The fistula tract 161 is a tube like structure internally within the body. Fig. 5c also shows the needle 128 in its unlocked position. The feeler tube 162 is prevented from moving into tissue 164 because it is unable to pierce the tissue 164. For example, the feeler tube 162 may be too soft and/or too blunt to pierce the tissue 164. The tissue 164 causes the feeler tube 162 to move, e.g. by means of a spring (not shown) towards the proximal end of the injection device. However, the needle 128 continues to advance to its unlocked position, i.e. its extended position, allowing injection to take place as described above in relation to Figs. 1 to 4b.

Fig. 5d shows advancement of the needle 128 where the needle 128 is located in a cavity 166 in the tissue 164. As opposed to the operation of the injection device 100 in Fig. 5c, the feeler tube 162 has nothing to block its path, and it moves with the needle 128. Thus, the needle 128 is not exposed from the feeler tube 162, and remains in its locked position and injection will not take place.

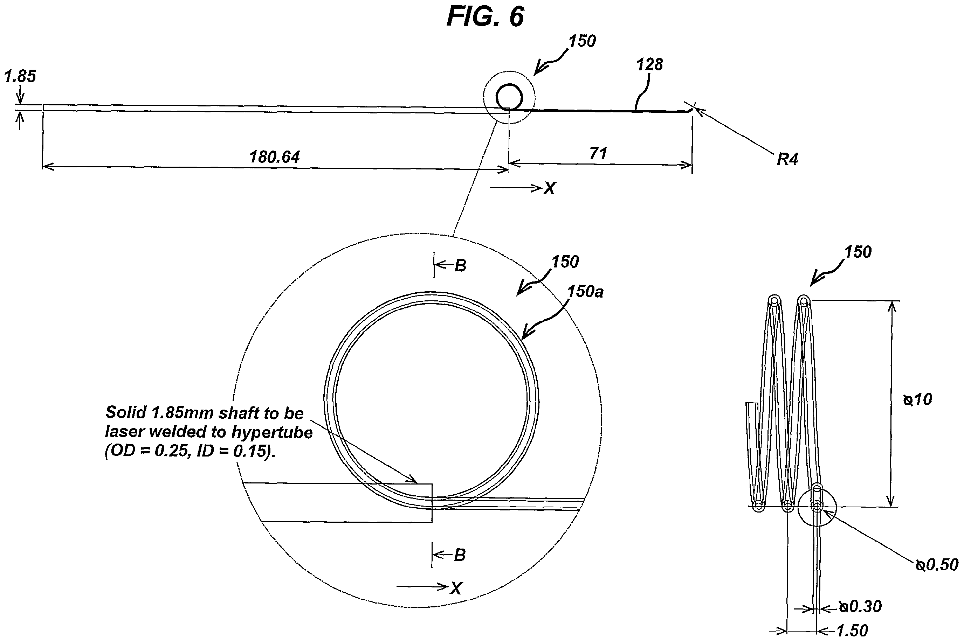

Fig. 6 shows the flexible fluid pathway 150 in more detail. The flexible fluid pathway 150 is, in one embodiment, manufactured as a helical coil of hollow tubing 151a, which can extend and contract in diameter as the needle 128 moves relative to the syringe 120, thereby permitting the needle 128 to move along the direction X. The coil is formed from a resilient, flexible metallic material.

It will of course be understood that the present invention has been described above purely by way of example and modifications of detail can be made within the scope of the invention.

## Claims

1. An injection device (100) comprising:
a housing (102) adapted to receive a fluid container (120) and hold the fluid container stationary relative to the housing during use of the device;
a hollow catheter connected to a distal end of the housing wherein the hollow catheter comprises an exit aperture at its distal end;
a plunger (106) adapted to discharge fluid from the fluid container,
a discharge nozzle (128) movable between a first retracted position in which the discharge nozzle is contained within the catheter and does not project from the exit aperture of the catheter, and a first extended position in which the discharge nozzle has moved relative to the fluid container and extends from the housing through the exit aperture of the catheter, wherein the discharge nozzle is a needle,
wherein the fluid container and discharge nozzle are in fluid communication with each other in the first extended position; and
a drive adapted to advance the discharge nozzle from its first retracted position to its first extended position and further adapted to act upon the plunger to expel fluid from the fluid container through the discharge nozzle, such that only when the discharge nozzle has reached its first extended position, the drive acts upon the plunger to expel fluid from the fluid container through the discharge nozzle,
**characterized in that** the plunger comprises a serrated ratchet and **in that** the drive comprises:
a first member (144) which is connected to the discharge nozzle;
a second member (146) which comprises a resiliently biased member adapted to engage the ratchet at a predetermined position of the drive relative to the housing; and
a retainer (147) which prevents the second drive member from engaging with the serrated ratchet for a predetermined distance.

2. The injection device of claim 1, wherein the drive is adapted to act upon the discharge nozzle and the plunger such that the discharge nozzle and plunger each move a predetermined distance in a single continuous motion.

3. The injection device of any preceding claim, further comprising a releasable stop means (148) for releasably stopping the plunger after it has been moved a predetermined distance.

4. The injection device of any preceding claim, wherein the fluid container comprises a cannular body, a flange at one end of the body and a discharge nozzle at a distal end of the body.

5. The injection device of any preceding claim, further comprising a flexible fluid pathway (150) between the fluid container and the discharge nozzle.

6. The injection device of claim 5, wherein the flexible pathway is adapted to straighten and bend, or extend and contract such that the discharge nozzle is movable between its first retracted position and its first extended position.

7. The injection device of any preceding claim, wherein when the discharge nozzle has reached its first extended position, it continues to extend a predetermined distance as the drive is moved.

8. The injection device of any one of claim 3 to 7, wherein when the discharge nozzle is at its first extended position, the discharge nozzle is at an angle to the first sleeve.

9. The injection device of claim 1 further comprising:
a sleeve (162) surrounding at least a portion of the discharge nozzle,
wherein the sleeve is adapted to move with the discharge nozzle as the discharge nozzle is being extended into its first extended position, and the sleeve is further adapted to move a predetermined distance relative to the discharge nozzle to expose a section of the discharge nozzle from an end of the sleeve.

10. The injection device of claim 9, wherein the drive is adapted to eject fluid from the fluid container and discharge nozzle only when the sleeve has moved the predetermined distance.

11. The injection device of claim 9 or claim 10, further comprising an indicator connected to the sleeve and discharge nozzle which is viewable by a user of the injection device and which is adapted to indicate that fluid can be ejected from the fluid container only when the sleeve is displaced relative to the discharge nozzle.

12. The injection device of claim 9 or claim 10, further comprising a locking mechanism which is connected to the sleeve, discharge nozzle and drive and which is adapted to permit the drive to be moved to eject fluid from the fluid container only when the sleeve is displaced relative to the discharge nozzle.

13. The injection device of any one of claims 9 to 12, wherein the sleeve and discharge nozzle move relative to each other when the sleeve and discharge nozzle come into contact with tissue, such that the discharge nozzle enters the tissue and the sleeve does not enter the tissue.

14. A kit, comprising the injection device of any preceding claim and a fluid container comprising a population of cells.

## Patentansprüche

1. Injektionsvorrichtung (100) umfassend:
ein Gehäuse (102) angepasst zum Aufnehmen eines Flüssigkeitsbehälters (120) und zum Festhalten des Flüssigkeitsbehälters in Bezug auf das Gehäuse während der Benutzung der Vorrichtung;
ein hohler Katheter verbunden mit einem distalen Ende des Gehäuses, wobei der hohle Katheter an seinem distalen Ende eine Austrittsöffnung umfasst;
ein Kolben (106) angepasst zum Abgeben von Flüssigkeit aus dem Flüssigkeitsbehälter;
eine Abgabedüse (128) bewegbar zwischen einer ersten zurückgezogenen Position, in der sich die Abgabedüse im Katheter befindet und nicht aus der Austrittsöffnung des Katheters herausragt, und einer ersten ausgefahrenen Position, in der die Abgabedüse sich relativ zu dem Flüssigkeitsbehälter bewegt hat und durch die Austrittsöffnung des Katheters aus dem Gehäuse herausragt, wobei die Abgabedüse eine Kanüle ist,
wobei der Flüssigkeitsbehälter und die Abgabedüse in der ersten ausgefahrenen Position miteinander im flüssigen Austausch stehen; und
ein Antrieb angepasst zum Vorantreiben der Abgabedüse von ihrer ersten zurückgezogenen Position zu ihrer ersten ausgefahrenen Position und des Weiteren angepasst, um auf den Kolben einzuwirken, um Flüssigkeit vom Flüssigkeitsbehälter durch die Abgabedüse abzugeben, so dass der Antrieb nur dann auf den Kolben zum Abgeben von Flüssigkeit vom Flüssigkeitsbehälter durch die Abgabedüse einwirkt, wenn die Abgabedüse ihre erste ausgefahrene Position erreicht hat,
**dadurch gekennzeichnet, dass** der Kolben eine gezahnte Ratsche umfasst und dass der Antrieb umfasst:
ein erstes Element (144), das mit der Abgabedüse verbunden ist;
ein zweites Element (146), das ein elastisch vorgespanntes Element umfasst, das angepasst ist, die Ratsche an einer vorbestimmten Position des Antriebs relativ zum Gehäuse einzurasten; und
einen Halter (147), der für eine vorgegebene Strecke verhindert, dass das zweite Antriebselement in die gezahnte Ratsche einrastet.

2. Injektionsvorrichtung nach Anspruch 1, wobei der Antrieb angepasst ist, um auf die Abgabedüse und den Kolben einzuwirken, so dass sich die Abgabedüse und der Kolben jeweils in einer einzigen kontinuierlichen Bewegung über eine vorgegebene Strecke bewegen.

3. Injektionsvorrichtung nach einem der vorhergehenden Ansprüche, weiter umfassend einen lösbaren Anschlag (148) zum lösbaren Anhalten des Kolbens, nachdem er über eine vorgegebene Strecke bewegt wurde.

4. Injektionsvorrichtung nach einem der vorhergehenden Ansprüche, wobei der Flüssigkeitsbehälter einen Kanülenkörper, einen Flansch an einem Ende des Körpers und eine Abgabedüse am distalen Ende des Körpers umfasst.

5. Injektionsvorrichtung nach einem der vorhergehenden Ansprüche, weiter umfassend eine flexible Flüssigkeitsleitung (150) zwischen dem Flüssigkeitsbehälter und der Abgabedüse.

6. Injektionsvorrichtung nach Anspruch 5, wobei die flexible Leitung angepasst ist zum Geradewerden und Verbiegen, oder Ausstrecken und Zusammenziehen, so dass die Abgabedüse zwischen ihrer ersten zurückgezogenen Position und ihrer ersten ausgefahrenen Position bewegbar ist.

7. Injektionsvorrichtung nach einem der vorhergehenden Ansprüche, wobei sich die Abgabedüse, wenn sie ihre erste ausgefahrene Position erreicht hat, weiter über eine vorgegebene Strecke ausstreckt, während der Antrieb bewegt wird.

8. Injektionsvorrichtung nach einem der Ansprüche 3 bis 7, wobei die Abgabedüse einen Winkel mit der ersten Hülse bildet, wenn sie in ihrer ersten ausgefahrenen Position ist.

9. Injektionsvorrichtung nach Anspruch 1, weiter umfassend:
eine Hülse (162), die mindestens einen Teil der Abgabedüse umgibt,
wobei die Hülse angepasst ist zur Bewegung mit der Abgabedüse, wenn die Abgabedüse in ihre erste ausgefahrene Position ausgefahren wird, und die Hülse weiter angepasst ist zur Bewegung über eine vorgegebene Strecke relativ zur Abgabedüse, um einen Teil der Abgabedüse an einem Ende der Hülse freizulegen.

10. Injektionsvorrichtung nach Anspruch 9, wobei der Antrieb angepasst ist, um Flüssigkeit aus dem Flüssigkeitsbehälter und der Abgabedüse nur dann abzugeben, wenn die Hülse sich über die vorgegebene Strecke bewegt hat.

11. Injektionsvorrichtung nach Anspruch 9 oder 10, weiter umfassend eine mit dem Gehäuse und der Abgabedüse verbundene Anzeige, die von einem Nutzer der Injektionsvorrichtung gesehen werden kann und die angepasst ist, um nur dann anzuzeigen, dass Flüssigkeit vom Flüssigkeitsbehälter ausgestoßen werden kann, wenn die Hülse im Vergleich zur Abgabedüse verschoben ist.

12. Injektionsvorrichtung nach Anspruch 9 oder 10, weiter umfassend einen Verschlussmechanismus, der mit der Hülse, der Abgabedüse und dem Antrieb verbunden ist, und der angepasst ist. um die Bewegung des Antriebs zum Abgeben von Flüssigkeit aus dem Flüssigkeitsbehälter nur dann zu erlauben, wenn die Hülse im Vergleich zur Abgabedüse verschoben ist.

13. Injektionsvorrichtung nach einem der Ansprüche 9 bis 12, wobei die Hülse und die Abgabedüse sich relativ zueinander bewegen, wenn die Hülse und die Abgabedüse mit Gewebe in Kontakt kommen, so dass die Abgabedüse in das Gewebe eindringt und die Hülse nicht in das Gewebe eindringt.

14. Kit, umfassend die Injektionsvorrichtung nach einem der vorhergehenden Ansprüche und einen Flüssigkeitsbehälter umfassend eine Zellpopulation.

## Revendications

1. Dispositif d'injection (100) comprenant :
un logement (102) apte à recevoir un récipient de fluide (120) et à maintenir le récipient de fluide fixe par rapport au logement pendant l'utilisation du dispositif ;
un cathéter creux connecté à une extrémité distale du logement, sachant que le cathéter creux comprend une ouverture de sortie à son extrémité distale ;
un plongeur (106) apte à évacuer du fluide du récipient de fluide,
une buse de décharge (128) mobile entre une première position rétractée dans laquelle la buse de décharge est contenue à l'intérieur du cathéter et ne dépasse pas de l'ouverture de sortie du cathéter, et une première position étendue dans laquelle la buse de décharge s'est déplacée par rapport au récipient de fluide et s'étend depuis le logement à travers l'ouverture de sortie du cathéter, sachant que la buse de décharge est une aiguille,
sachant que le récipient de fluide et la buse de décharge sont en communication fluidique l'un avec l'autre dans la première position étendue ; et
un entraîneur apte à avancer la buse de décharge de sa première position rétractée à sa première position étendue et en outre apte à agir sur le plongeur pour expulser du fluide du récipient de fluide via la buse de décharge, de sorte que l'entraîneur agisse sur le plongeur pour expulser du fluide du récipient de fluide via la buse de décharge seulement lorsque la buse de décharge a atteint sa première position étendue,
**caractérisé en ce que** le plongeur comprend un cliquet cranté et **en ce que** l'entraîneur comprend :
un premier élément (144) qui est connecté à la buse de décharge ;
un deuxième élément (146) qui comprend un élément actionné de manière souple, apte à mettre en prise le cliquet dans une position prédéterminée de l'entraîneur par rapport au logement ; et
un organe de retenue (147) qui empêche le deuxième élément d'entraîneur de se mettre en prise avec le cliquet cranté sur une distance prédéterminée.

2. Le dispositif d'injection de la revendication 1, sachant que l'entraîneur est apte à agir sur la buse de décharge et le plongeur de sorte que la buse de décharge et le plongeur se déplacent chacun d'une distance prédéterminée en un même mouvement continu.

3. Le dispositif d'injection d'une quelconque revendication précédente, comprenant en outre des moyens d'arrêt (148) amovibles pour arrêter de manière amovible le plongeur après qu'il a été déplacé d'une distance prédéterminée.

4. Le dispositif d'injection d'une quelconque revendication précédente, sachant que le récipient de fluide comprend un corps canulaire, une flasque à une extrémité du corps et une buse de décharge à une extrémité distale du corps.

5. Le dispositif d'injection d'une quelconque revendication précédente, comprenant en outre une voie de passage de fluide (150) flexible entre le récipient de fluide et la buse de décharge.

6. Le dispositif d'injection de la revendication 5, sachant que la voie de passage flexible est apte à se redresser et se plier, ou s'étendre et se contracter de sorte que la buse de décharge soit mobile entre sa première position rétractée et sa première position étendue.

7. Le dispositif d'injection d'une quelconque revendication précédente, sachant que lorsque la buse de décharge a atteint sa première position étendue, elle continue de s'étendre d'une distance prédéterminée tandis que l'entraîneur est déplacé.

8. Le dispositif d'injection de l'une quelconque des revendications 3 à 7, sachant que lorsque la buse de décharge est dans sa première position étendue, la buse de décharge se trouve à un angle par rapport au premier manchon.

9. Le dispositif d'injection de la revendication 1, comprenant en outre :
un manchon (162) entourant au moins une partie de la buse de décharge,
sachant que le manchon est apte à se déplacer avec la buse de décharge tandis que la buse de décharge est en train de s'étendre dans sa première position étendue, et le manchon est en outre apte à se déplacer d'une distance prédéterminée par rapport à la buse de décharge pour exposer une section de la buse de décharge par rapport à une extrémité du manchon.

10. Le dispositif d'injection de la revendication 9, sachant que l'entraîneur est apte à éjecter du fluide du récipient de fluide et de la buse de décharge seulement lorsque le manchon s'est déplacé de la distance prédéterminée.

11. Le dispositif d'injection de la revendication 9 ou de la revendication 10, comprenant en outre un indicateur connecté au manchon et à la buse de décharge, qui est visualisable par un utilisateur du dispositif d'injection et qui est apte à indiquer que du fluide peut être éjecté du récipient de fluide seulement lorsque le manchon est déplacé par rapport à la buse de décharge.

12. Le dispositif d'injection de la revendication 9 ou de la revendication 10, comprenant en outre un mécanisme de verrouillage qui est connecté au manchon, à la buse de décharge et à l'entraîneur et qui est apte à permettre à l'entraîneur de se déplacer pour éjecter du fluide du récipient de fluide seulement lorsque le manchon est déplacé par rapport à la buse de décharge.

13. Le dispositif d'injection de l'une quelconque des revendications 9 à 12, sachant que le manchon et la buse de décharge se déplacent l'un par rapport à l'autre lorsque le manchon et la buse de décharge entrent en contact avec du tissu, de sorte que la buse de décharge entre dans le tissu et le manchon n'entre pas dans le tissu.

14. Kit, comprenant le dispositif d'injection d'une quelconque revendication précédente et un récipient de fluide comprenant une population de cellules.
